Europäisches Patentamt

European Patent Office

Office européen des brevets

(19)

(11) EP 0 623 629 B1

(12) **FASCICULE DE BREVET EUROPEEN**

(45) Date de publication et mention
de la délivrance du brevet:
**21.08.1996 Bulletin 1996/34**

(51) Int Cl.$^6$: **C08B 37/10**

(21) Numéro de dépôt: **94400994.3**

(22) Date de dépôt: **06.05.1994**

(54) **Fractions d'héparine purifiées, procédé d'obtention et compositions pharmaceutiques les contenant**

Gereinigte Heparin Fraktionen, Verfahren zur Herstellung und diese enthaltende pharmazeutische Zusammensetzungen

Purified heparin fractions, process for obtaining them and pharmaceutical compositions containing the same

(84) Etats contractants désignés:
**AT BE CH DE DK ES FR GB GR IE IT LI LU MC NL PT SE**

(30) Priorité: **07.05.1993 FR 9305534**

(43) Date de publication de la demande:
**09.11.1994 Bulletin 1994/45**

(73) Titulaire: **CHOAY S.A.**
**F-75008 Paris (FR)**

(72) Inventeurs:
• **Branellec, Jean-François**
**F-76130 Mont Saint Aignan (FR)**
• **Espejo, José**
**F-76230 Bois Guillaume (FR)**
• **Picart, Philippe**
**F-76000 Rouen (FR)**

(74) Mandataire: **Gillard, Marie-Louise et al**
**Cabinet Beau de Loménie**
**158, rue de l'Université**
**75340 Paris Cédex 07 (FR)**

(56) Documents cités:
**EP-A- 0 037 319       WO-A-82/03627**

• **WATER SUPPLY vol. 6 , 1988 , GB pages 199 - 205 P. PRINCZ ET AL. 'Photochemical nitrate removal from drinking water'**

EP 0 623 629 B1

## Description

La présente invention concerne des fractions d'héparine purifiées et plus précisément des fractions d'héparine exemptes de groupes N-NO, un procédé pour leur préparation et des médicaments les contenant.

Plus particulièrement, la présente invention se rapporte à des fractions d'héparine, obtenues par dépolymérisation nitreuse, pratiquement dépourvues de composés nitrosés.

Il est connu que des fractions d'héparine sont obtenues par dépolymérisation nitreuse, comme décrit par exemple dans les demandes de brevets EP-A-0014184 et EP-A-0027089. Certaines fractions d'héparine, préparées par dépolymérisation nitreuse, sont des principes actifs de médicaments qui sont entrés dans l'usage commun sous l'appellation "héparines de bas poids moléculaire" ou "héparines de faible masse moléculaire". Pour tout détail concernant les héparines de bas poids moléculaire utilisées comme médicaments, leur titrage et leur degré de sulfatation, exprimé par le rapport sulfates/carboxyles, on peut se référer à la publication "Héparines de faible masse moléculaire". Pharmeuropa, Octobre 1991, 3, N. 3, pp. 161-165, ainsi qu'à la monographie proposée pour la Pharmacopée Européenne "Heparina massae molecularis minoris", Février 1993 (PA/PH/Exp. 3T (92) 93). Des héparines de bas poids moléculaire ainsi préparées et utiles comme principes actifs de médicaments sont décrites dans les demandes de brevet ou brevets publiés sous les numéros FR-A-2478646, EP-A-0037319, EP-A-0076279, EP-A-0181259. Elles sont connues sous les Dénominations Communes Internationales (DCI) nadroparine calcique, daltéparine sodique, reviparine sodique et constituent des principes actifs de spécialités commercialisées ou potentielles. Notamment, la reviparine sodique constitue le principe actif de la spécialité CLIVARIN® commercialisée en Allemagne. D'autres héparines de bas poids moléculaire obtenues par dépolymérisation nitreuse sont également présentes dans des spécialités mises à la disposition du corps médical en Allemagne (MONO-EMBOLEX® NM), en Autriche (SANDOPARIN® et TROPARIN®), et en Suisse (SANDOPARINE®).

Le procédé de dépolymérisation nitreuse comporte la formation de composés nitrosés non volatils (ci-après également appelés composés N-NO ou tout simplement N-NO) par addition d'un groupe -NO à des composants nitrosables présents dans l'héparine ou dans ses fractions ou fragments. Bien que la quantité de composés nitrosés présents dans les fractions d'héparine soit très faible et ne porte pas de préjudice à leur utilisation en tant que médicaments, puisqu'il s'agit de composés non volatils, la fabrication de ces produits à l'échelle industrielle comporte la manipulation de grandes quantités de poudres. Leur élimination totale ou pratiquement totale pourrait donc apparaître utile pour parfaire les caractéristiques des héparines de bas poids moléculaire qui sont utilisées comme principes actifs de spécialités pharmaceutiques.

Dans le cas d'héparines de bas poids moléculaire dont la masse moléculaire moyenne est faible, par exemple dans le cas d'un produit qui sera dénommé ci-après CY 222 (masse moléculaire moyenne de 1.700 Da à 3.300 Da), la quantité de composés nitrosés peut être supérieure à celle des produits ayant une masse moléculaire plus élevée. Ce produit répond à la définition des produits revendiqués dans les demandes de brevet et brevet publiés sous les numéros FR-2 478 646 et EP-0 037 319, et peut être préparé selon les procédés décrits dans ces mêmes demandes de brevet ou brevet.

Il est donc hautement souhaitable, pour les raisons énoncées ci-dessus, de pouvoir disposer de fractions d'héparine obtenues par dépolymérisation nitreuse et pratiquement dépourvues de composés nitrosés.

Un procédé d'élimination des nitrates dans l'eau potable avec stérilisation simultanée de l'eau, par irradiation aux rayons ultraviolets à une longueur d'onde inférieure à 200 nm, de préférence 185 nm, et à un pH basique est décrit dans l'article P.PRINCZ et al., Water Supply, 1988, 6, 199-205. Néanmoins, ce document concernant le traitement de l'eau ne permettait pas de prévoir si une irradiation aux rayons ultraviolets était applicable à un produit biologiquement actif, sans entraîner d'effets néfastes sur ses propriétés biologiques et/ou physicochimiques.

Il a été maintenant trouvé qu'en soumettant une fraction d'héparine obtenue par dépolymérisation nitreuse à l'action des rayons ultraviolets (ci-après désignés simplement UV), on peut éliminer presque totalement les composés nitrosés présents, et on obtient une fraction d'héparine purifiée ayant un taux de groupes N-NO égal voire inférieur à celui de l'héparine naturelle.

Il a été également trouvé que dans les conditions utilisées, le rayonnement UV ne modifie pas la structure des fractions d'héparine, qui conservent toutes leurs propriétés biologiques et physicochimiques.

Ainsi, selon un de ses aspects, la présente invention concerne des fractions d'héparine obtenues par dépolymérisation nitreuse ayant un taux en groupes N-NO inférieur ou égal à 500 parties par billion (ppb), avantageusement inférieur ou égal à 150 ppb, de préférence inférieur ou également à 100 ppb, de préférence encore à 50 ppb, le taux de 50 ppb étant la quantité minimale quantifiable dans l'état actuel des connaissances. Lesdites fractions sont de préférence des héparines de bas poids moléculaire.

Selon un aspect avantageux, l'invention concerne une héparine de bas poids moléculaire purifiée obtenue par dépolymérisation nitreuse, à savoir une héparine dépolymérisée obtenue par dépolymérisation nitreuse d'héparine d'origine naturelle de préférence provenant du mucus intestinal de porc, du poumon de boeuf ou toute autre héparine extraite des tissus ou des organes d'animaux divers ayant les caractéristiques suivantes :

- une masse moléculaire moyenne inférieure à 8.000 Da,
- au moins 60 % de tous les constituants ont une masse moléculaire inférieure à 8.000 Da,
- activité anti-facteur Xa pas inférieure à 60 UI/mg,
- rapport activité anti-facteur Xa/anti-facteur 11a pas inférieur à 1,5,
- un taux en groupes N-NO égal ou inférieur à 500 ppb, avantageusement à 150 ppb, de préférence à 100 ppb, de préférence encore à 50 ppb, ladite héparine dépolymérisée étant sous forme de sel pharmaceutiquement acceptable, de préférence de sodium ou de calcium.

L'activité anti facteur Xa et le rapport d'activité anti-facteur Xa/anti-facteur IIa sont évalués par rapport à l'étalon international des héparines de bas poids moléculaire ; référence OMS 1-85/600.

La masse moléculaire moyenne ci-dessus est déterminée selon la Monographie proposée pour la Pharmacopée européenne (voir référence ci-dessus).

Dans la présente description, on utilise le terme "exemptes de groupes N-NO" pour qualifier les fractions d'héparine ou les héparines de bas poids moléculaire obtenues par dépolymérisation nitreuse et contenant au plus 500 ppb, avantageusement au plus 150 ppb, notamment au plus 100 ppb, de préférence au plus 50 ppb de groupes N-NO. Sous le terme "constituants", on désigne l'ensemble des molécules à partir desquelles est constituée l'héparine de bas poids moléculaire.

Les héparines de bas poids moléculaire exemptes de groupes N-NO, objet de la présente invention, ont dans la majorité de leurs constituants, une structure 2-O-sulfo-$\alpha$-L-idopyranosuronique à l'extrémité non réductrice, et une structure 6-O-sulfo-2,5-anhydro-D-mannitol à l'extrémité réductrice et un degré de sulfatation qui ne diffère pas sensiblement de celui de l'héparine d'origine naturelle non fractionnée. Ce degré de sulfatation est compris entre 2 et 2,5, de préférence entre 2,0 et 2,3.

Des héparines de bas poids moléculaire, avantageuses selon la présente invention, ont des distributions de masses moléculaires variables, la masse moléculaire de 90% de leurs constituants s'étalant entre 2.000 Da et 10.000 Da, de préférence entre 2.000 Da et 9.000 Da, avantageusement entre 2.000 Da et 8.000 Da. Par ailleurs, ces héparines ont une masse moléculaire majoritaire située entre 3.000 Da et 6.000 Da, de préférence entre 4.000 Da et 5.000 Da.

Une autre héparine de bas poids moléculaire préférée selon l'invention a une masse moléculaire majoritaire s'étalant entre 1.700 Da et 3.300 Da, la masse moléculaire de 90 % de tous les constituants s'étalant entre 1.000 Da et 8.000 Da.

Sous le terme "masse moléculaire majoritaire", on désigne la masse moléculaire des constituants de l'héparine qui correspondent au sommet du profil chromatographique obtenu par chromatographie d'exclusion, en utilisant un détecteur UV à $\lambda = 205$ nm.

Le CY 222 purifié, défini comme sel de sodium d'une héparine dépolymérisée obtenue par dépolymérisation à l'acide nitreux de l'héparine d'origine naturelle de mucus intestinal de porc ayant :

- une masse moléculaire majoritaire s'étalant entre 1.700 Da et 3.300 Da, 90 % des constituants ayant une masse moléculaire s'étalant entre 1.000 Da et 8.000 Da,
- une structure 2-O-sulfo-$\alpha$-L-idopyranosuronique à l'extrémité non réductrice et une structure 6-O-sulfo-2,5-anhydro-D-mannitol à l'extrémité réductrice de la majorité de ses constituants,
- un degré de sulfatation d'environ 2,1,
- une activité anti-facteur Xa de 60-80 UI/mg,
- une activité anti-facteur IIa pas supérieure à 25 UI/mg, plus précisément de 10-15 UI/mg,
- un taux en groupes N-NO inférieur ou égal à 100 ppb ($1.10^{-7}$ mg de composés nitrosés par mg de produit), de préférence inférieur ou égal à 50 ppb ($5.10^{-8}$ mg de composés nitrosés par mg de produit), représente une fraction d'héparine préférée selon la présente invention.

Les fractions d'héparine exemptes de groupes N-NO (héparines de bas poids moléculaire) de la présente invention, sont préparées selon un autre aspect de la présente invention.

Ainsi, la présente invention concerne également un procédé pour la préparation de fractions d'héparine contenant au plus 500 ppb de groupes N-NO, caractérisé en ce qu'on soumet une solution aqueuse d'une fraction d'héparine obtenue par dépolymérisation nitreuse à un rayonnement ultraviolet.

Comme produit de départ on peut utiliser toute fraction d'héparine obtenue par dépolymérisation nitreuse. Un tel produit de départ contient en général un taux de groupes N-NO situé entre 1.000 et 20.000 ppb.

La détermination des groupes N-NO est effectuée par la méthode de B. Pignatelli et al., décrite dans Analyst, September 1989, 114, pp. 1103-1108 et dans Analyst, July 1987, 112, pp. 945-949 adaptée à l'héparine et à ses fractions, de préférence aux héparines de bas poids moléculaire.

Le procédé de la présente invention peut être conduit sur une héparine de bas poids moléculaire de qualité pharmaceutique, dissoute dans l'eau, ou bien pendant la fabrication industrielle, après l'étape de dépolymérisation nitreuse et avant l'isolement du produit pur de qualité pharmaceutique. On utilise de préférence une héparine de bas poids moléculaire de qualité pharmaceutique.

Ainsi, comme matière première pour le procédé de la présente invention on peut utiliser n'importe quelle héparine de bas poids moléculaire obtenue par dépolymérisation nitreuse. Pour obtenir le maximum d'efficacité du procédé, il est avantageux d'utiliser des solutions d'héparine dépolymérisée exemptes de microparticules en suspension. La présence de ces microparticules peut-être due à une mauvaise dissolution de l'héparine dépolymérisée ou aux différentes impuretés ou encore aux restes de réactifs utilisés lors de la dépolymérisation ou lors de la purification. Les différentes solutions d'héparine dépolymérisée sont donc soumises de manière préférentielle à une filtration préalable.

Le procédé de la présente invention est extraordinairement efficace et souple, car il permet l'élimination de composés nitrosés de façon simple, rapide et sûre. En outre, le procédé de l'invention n'entraîne aucune modification dans la structure de la fraction d'héparine, dont les propriétés biologiques et les caractéristiques physicochimiques restent strictement identiques.

Le procédé de la présente invention peut être conduit en exposant une solution aqueuse à 5-15% m/V de fraction d'héparine obtenue par dépolymérisation nitreuse, de préférence d'héparine de bas poids moléculaire à purifier, sous un système de rayonnement UV à une longueur d'onde de 180 à 350 nm, de préférence à 254 nm, à une température de 5°C à 50°C, avantageusement de 15°C à 35°C, de préférence à température ambiante.

Le pH de la solution est légèrement acide ou légèrement basique, notamment entre 3 et 8, de préférence entre 5 et 8.

La durée du rayonnement dépend du système de rayonnement utilisé, de la puissance du rayonnement et de la quantité de groupes N-NO à éliminer, présents dans la fraction d'héparine. Elle est d'environ de 2 à 30 minutes, bien qu'après 9-15 minutes la purification soit en général quasiment complète.

Comme système de rayonnement UV, on peut envisager soit un système statique, soit un système dynamique permettant aux solutions de fractions de l'héparine de circuler autour de la source du rayonnement UV. On préfère les systèmes dynamiques. Dans ce dernier cas, la source du rayonnement UV peut être associée à un tube cylindrique (appareil de type "fermé") ou à un canal (appareil de type "ouvert"). Comme appareil de type "fermé", on peut utiliser le stérilisateur UV JR1-50 (KATADYN) ou tout autre appareil équivalent.

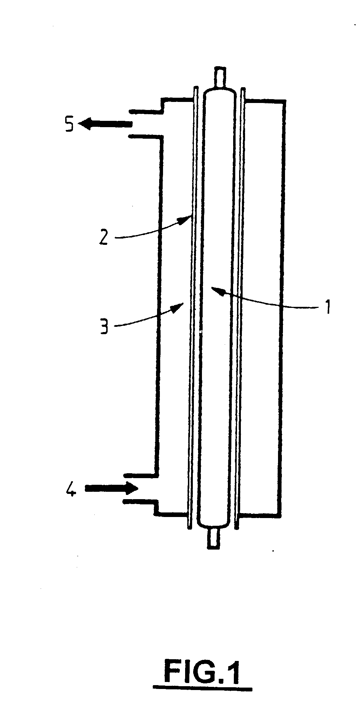

La Figure 1 représente en coupe un appareil de type "fermé". Cet appareil se compose d'une lampe UV (1) autour de laquelle est adaptée une gaine de quartz (2). Les solutions contenant les héparines de bas poids moléculaire circulent autour de la lampe UV protégée par la gaine de quartz (gaine du liquide circulant (3)). Elles sont introduites par une entrée (4) située au niveau de l'une des extrémités de la lampe UV, la sortie (5) étant située à l'autre extrémité de la lampe UV.

Lorsque l'on utilise un système dynamique, le débit des solutions de la fraction d'héparine avec lequel elles circulent autour de la source de rayonnement UV doit être ajusté de manière appropriée, pour obtenir la durée de rayonnement nécessaire pour éliminer les composés nitrosés. Les différents paramètres qui peuvent intervenir sont le diamètre de la veine du liquide circulant, la dimension et/ou la puissance de la source du rayonnement UV, la concentration de la solution aqueuse en fraction d'héparine et la quantité des composés nitrosés à éliminer contenue dans cette fraction.

La fraction d'héparine, ou, de préférence, l'héparine de bas poids moléculaire exempte de groupes N-NO ainsi obtenue, peut être récupérée selon les méthodes conventionnelles.

Lorsque comme produit de départ on utilise une héparine de bas poids moléculaire déjà de qualité pharmaceutique, il suffit de dissoudre le produit dans l'eau, d'ajuster le pH de la solution entre 3 et 8, de préférence entre 5 et 8 et de soumettre cette solution au procédé de la présente invention.

Comme produit de départ on peut également utiliser l'héparine d'origine naturelle non fractionnée, avantageusement sous la forme de sel, notamment l'héparine sodique, de préférence provenant du mucus intestinal de porc. Dans ce cas, le procédé de la présente invention constitue une étape dans la préparation du produit final purifié exempt de groupes N-NO, après la dépolymérisation nitreuse. Le procédé global partant de l'héparine, représente un objet ultérieur de la présente invention.

Ainsi, selon un autre de ses aspects, la présente invention concerne un procédé pour la préparation, par dépolymérisation nitreuse, d'une héparine de bas poids moléculaire ayant un taux en groupes N-NO inférieur ou égal à 500 ppb, avantageusement inférieur ou égal à 150 ppb, de préférence inférieur ou égal à 100 ppb, de préférence encore inférieur ou égal à 50 ppb, caractérisé en ce que :

(a) on traite une solution d'héparine sodique non fractionnée dans l'eau à 5-15 % m/V avec une solution d'un nitrite alcalin, de préférence le nitrite de sodium à raison de 15 à 69 g de nitrite par kg d'héparine mise en oeuvre, en présence d'acide chlorhydrique à un pH de 1 à 5, de préférence 1,5 à 4, pendant une durée de 20 à 50 minutes et on soumet le produit ainsi obtenu à une réduction, de préférence en milieu alcalin, avec 5-20 g de borohydrure de sodium par kg d'héparine de départ, on détruit l'excès de borohydrure de sodium par de l'acide chlorhydrique, on neutralise éventuellement le milieu réactionnel, et on précipite à l'éthanol, puis après avoir éventuellement soumis l'héparine de bas poids moléculaire ainsi obtenue à un ou plusieurs fractionnements alcooliques,

(b) on soumet la solution ainsi obtenue à un rayonnement ultraviolet,

(c) on procède éventuellement à une purification chromatographique et on isole l'héparine sodique dépolymérisée, purifiée et exempte de groupes N-NO par précipitation avec du chlorure de sodium et de l'éthanol, et, éventuellement,

(d) on transforme le sel de sodium en un autre sel pharmaceutiquement acceptable, le sel de calcium de préférence.

Selon un aspect préféré, la présente invention concerne un procédé pour la préparation du CY 222 purifié, défini comme sel de sodium d'une héparine dépolymérisée obtenue par dépolymérisation à l'acide nitreux de l'héparine d'origine naturelle de mucus intestinal de porc ayant :

- une masse moléculaire majoritaire s'étalant entre 1.700 Da et 3.300 Da,
- 90 % des constituants ayant une masse moléculaire s'étalant entre 1.000 Da et 8.000 Da,
- une structure 2-O-sulfo-$\alpha$-L-idopyranosuronique à l'extrémité non réductrice et une structure 6-O-sulfo-2,5-anhydro-D-mannitol à l'extrémité réductrice de la majorité de ses constituants,
- un degré de sulfatation d'environ 2,1,
- une activité anti-facteur Xa de 60-80 UI/mg,
- une activité anti-facteur IIa pas supérieure à 25 UI/mg,
- un contenu en groupes N-NO inférieur ou égal à 100 ppb, de préférence inférieur ou égal à 50 ppb, ledit procédé étant caractérisé en ce que :

(a) on traite une solution aqueuse d'héparine sodique d'origine naturelle non fractionnée avec de l'acide chlorhydrique et une quantité d'un nitrite alcalin de 3,5 à 4% en poids par rapport à l'héparine mise en oeuvre, tout en maintenant le pH acide et en surveillant la présence d'ions nitreux jusqu'à réaction négative, puis on alcalinise, on réduit avec du borohydrure de sodium et on isole en milieu neutre le produit dépolymérisé par précipitation par l'éthanol, puis,

(b) on fait passer une solution aqueuse du produit ainsi obtenu, à pH d'environ 7, sous un système à rayonnement ultraviolet à 254 nm, on introduit ensuite la solution ainsi obtenue au sommet d'une colonne échangeuse d'anions et, après rinçage de la colonne à l'eau et à pH d'environ 7, on récupère le produit final par précipitation avec du chlorure de sodium et d'éthanol.

A l'étape (a), on utilise dans les proportions décrites ci-dessus comme nitrite alcalin, le nitrite de sodium.

De préférence, dans l'étape (b) du procédé le système à rayonnement ultraviolet est muni d'une lampe de 16 W et la durée de l'exposition au rayonnement UV à 254 nm est de 9-15 minutes environ. On préfère un système de rayonnement UV dynamique et notamment un appareil de type "fermé". D'une manière avantageuse, la concentration des solutions soumises au rayonnement UV, est de 8-12 % m/V.

Selon un aspect ultérieur, le présente invention concerne des compositions pharmaceutiques contenant, en tant que principe actif, une fraction d'héparine purifiée, exempte de groupes N-NO, de préférence une héparine de bas poids moléculaire purifiée, exempte de composés nitrosés totaux, telle que décrite ci-dessus.

Pour les compositions selon la présente invention, la fraction d'héparine peut se présenter sous forme de lyophilisat ou en solution pour l'injection sous-cutanée, dans un solvant biologiquement acceptable stérile tel que l'eau ou dans une solution physiologique, dans des ampoules, dans des flacons, dans des seringues pré-remplies ou dans des dispositifs pour auto-injection du type "stylo". Chaque unité de compositions pharmaceutiques de dosage peut contenir de 50 à 50.000 UI anti-facteur Xa.

Les fractions d'héparine selon l'invention peuvent également être administrées par injection intraveineuse, seules ou en mélange avec d'autres principes actifs. Elles peuvent être encore administrées par nébulisation intranasale ou intrapulmonaire.

Une composition pharmaceutique préférée selon la présente invention contient, comme principe actif, le CY 222 exempt de groupes N-NO, tel que décrit ci-dessus dans des unités de dosage contenant de 10 mg à 5000 mg de CY 222 sous forme de sel de sodium.

Comme il a été indiqué précédemment, la détermination des groupes N-NO est effectuée par la méthode de B. Pignatelli et al, décrite dans Analyst, September 1989, 114, pp. 1103-1108 et dans Analyst, July 1987, 112, pp. 945-949, adaptée à l'héparine et aux héparines de bas poids moléculaire.

## Méthode analytique

Les produits à analyser sont de préférence en poudre, par conséquent, en cas de détermination des groupes N-NO présents dans une solution, on procède d'abord à une lyophilisation de ladite solution et on analyse après l'échantillon lyophilisé.

1- <u>Réactifs</u>

Les réactifs utilisés pour 100 mg de fraction d'héparine sont les suivants :

- Acétate d'éthyle traité à l'acide sulfamique 20% m/V, (une solution de 200 g d'acide sulfamique dans 1000 ml d'acétate d'éthyle agitée pendant 3 jours et filtrée sur papier avant utilisation),
- Acide bromhydrique à 15% dans l'acide acétique, (petits flacons ambrés avec bouchon en polyéthylène, contenant 5 ml d'acide bromhydrique à 30%, fermés sous argon et conservés à l'obscurité. Au moment de l'emploi on ajoute 5 ml d'acide acétique pur, on agite et on conserve dans un petit container à l'obscurité. On utilise 1 flacon par série de dosages),
- Formamide à 95% traité à l'acide sulfamique 5% m/V, (une solution de 1 g d'acide sulfamique dans 20 ml de formamide contenant 5% d'eau purifiée).

2- <u>Préparation des solutions étalons et de l'échantillon à analyser</u>

- Solution étalon de N-nitroso di-n-propylamine (NDPA)
  On injecte 78,62 g d'éthanol pur dans un flacon ISOPAC Sigma, contenant 1 g de N-nitroso di-n-propylamine, à travers le septum. La concentration est de 10000 ppm de NDPA (3379 ppm de N-NO). Puis on dilue au 1/100ème avec de l'éthanol pur et on aliquote en 0,5 ml dans des petits flacons capsulés. La concentration est de 100 ppm de NDPA. On conserve à l'obscurité et à +4°C.

    * *Solution étalon bas* : au moment de l'emploi on fait une dilution au 1/100ème dans l'éthanol puis au 1/17ème dans le formamide traité. La concentration sera de 0,0588 ppm de NDPA ou 19,9 ppb de N-NO.
    * *Solution étalon moyen* : au moment de l'emploi on fait une dilution au 1/61ème dans l'éthanol puis au 1/11ème dans le formamide traité. La concentration sera de 0,149 ppm de NDPA ou 50,4 ppb de N-NO.
    * *Solution étalon haut* : au moment de l'emploi on fait une dilution au 1/10ème dans l'éthanol puis au 1/10ème dans le formamide traité. La concentration sera de 1 ppm de NDPA ou 338 ppb de N-NO.

- Echantillon à analyser
  On sèche les échantillons de fraction d'héparine 12 heures à 60°C sous vide, sous anhydride phosphorique. On dissout 100 mg de fraction d'héparine dans 1 ml de formamide traité et on agite 30 minutes sur un agitateur-secoueur.

3- <u>Appareillage utilisé</u>

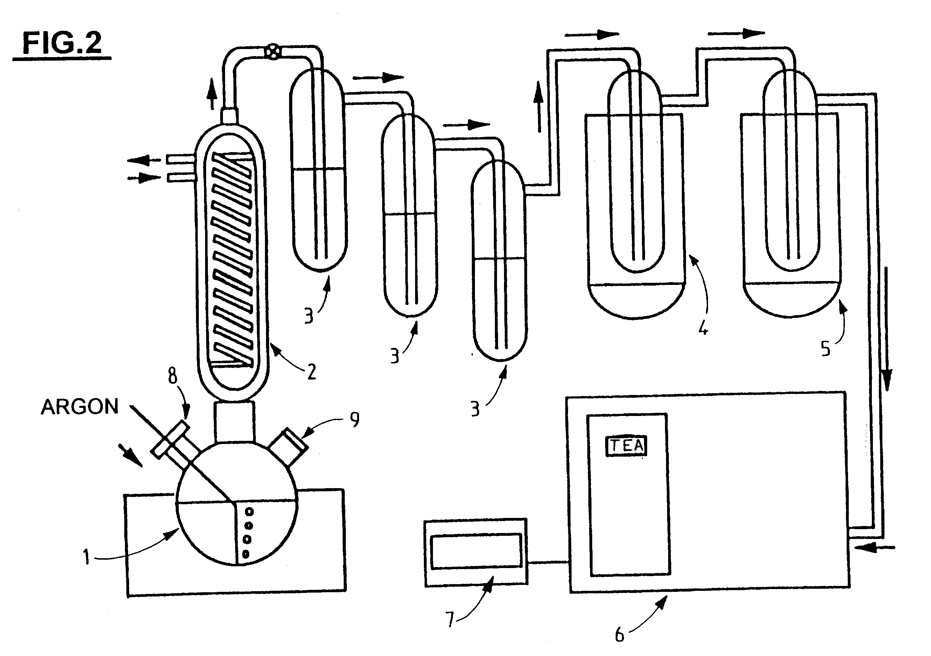

L'appareillage utilisé est illustré dans la Figure 2.

Montage

L'appareil se compose d'un ballon de 500 ml en pyrex (1) surmonté d'un réfrigérant à double effet (2) refroidi à -15°C. Celui-ci est relié à trois barboteurs montés en série (3) contenant chacun 30 ml d'hydroxyde de sodium à 30%. Ces derniers sont reliés à deux pièges à froid montés en série, l'un préparé avec de l'éthanol en fusion à -120°C (4) et l'autre avec de l'isopentane en fusion à -160°C (5). Ceux-ci sont reliés, enfin, à un détecteur à chimiluminescence (TEA) Model 610 (Thermedics Detection INC.) ou équivalent (6). Le détecteur est relié à un intégrateur/enregistreur (7).
Le ballon est équipé d'un côté, d'un bouchon percé (8) permettant d'introduire une canule par laquelle arrive un flux d'argon,et de l'autre côté, d'un rodage à vis muni d'un bouchon permettant de fixer un septum type CPG (9) par lequel on injecte l'échantillon. C'est le flux d'argon et le vide produit par la pompe à vide du TEA qui entraînent les gaz vers le TEA.

4- <u>Protocole opératoire</u>

4.1- Réglage du détecteur

Le TEA est réglé selon les instructions du fabricant afin d'obtenir la sensibilité et la reproductibilité maximales.
Une heure avant le dosage, on ouvre l'oxygène, pression 2 bars, on règle le débit à 0,02 sur le débitmètre du TEA 610 (Thermedics Detection INC.).

4.2- Préparation des pièges à soude

On remplit chaque piège avec 30 ml d'hydroxyde de sodium à 30%.

4.3- Préparation des pièges à froid

*Piège à -120°C* : Dans un Dewar contenant 250 ml d'éthanol, on verse doucement, en agitant à l'aide d'une spatule de bois, de l'azote liquide jusqu'à l'obtention d'une pâte.
*Piège à -160°C* : Dans un Dewar contenant 250 ml d'isopentane, on verse doucement, en agitant à l'aide d'une spatule de bois, de l'azote liquide jusqu'à l'obtention d'une pâte.
Les deux pièges en verre sont placés dans leurs Dewars respectifs et branchés en série sur le circuit.

4.4- Déshydratation de l'ensemble ballon-réfrigérant

On met à reflux 50 ml d'acétate d'éthyle pendant 1 heure, sous argon, sans brancher au TEA.

4.5- Réaction et dosage

Dans un nouveau ballon propre et sec, équipé d'un septum, on introduit 30 ml d'acétate d'éthyle traité, on monte celui-ci sous le réfrigérant (le cryostat doit être mis en route 2 heures avant à -15°C), on place le chauffe-ballon et met en chauffe (position 4). On branche la canule d'argon, règle le débit à 0,1 l/minute et vérifie l'étanchéité de tout le circuit. Seul le branchement sur le TEA reste ouvert pour éviter une surpression. Lorsque l'acétate d'éthyle est à reflux, on passe sous vide très doucement et en même temps on resserre l'arrivée sur le TEA. Le vide se fait dans tout le circuit et atteint 2-4 mm de Hg lorsque le système est équilibré. On règle le zéro du TEA à 10% pleine échelle de l'intégrateur/enregistreur.
A travers le septum, on injecte successivement 0,5 ml d'eau purifiée, 2 ml d'acide bromhydrique dilué puis à nouveau 2 ml d'acide bromhydrique dilué, entre chaque injection on vérifie le retour en ligne de base sur l'intégrateur/enregistreur. Une fois les réactifs injectés dans le ballon, on attend le retour en ligne de base et injecte 50 µl d'étalon ou d'échantillon. Entre chaque injection, on attend le retour en ligne de base. Lors des dosages, on encadre 5 échantillons par 2 étalons et on procède de façon à ce que la manipulation complète ne dure pas plus de 60 minutes.

4.6- Evaluation de la quantité de groupes N-NO

La quantité de N-NO, en ppb, est calculée par la formule :

$$\text{ppb en N-NO} = \frac{\text{surface échantillon} \times Cs \times 0,05 \times 44 \times 1000}{\text{surface étalon} \times 0,05 \times 0,1 \times 130,2}$$

où

- Cs : concentration de l'étalon en ppm de NDPA (0,0588 ou 0,149 ou 1 ppm)
- 0,05 : (numérateur) prise d'essai de l'étalon (ml)
- 44 : masse moléculaire de N-NO
- 1000 : conversion ppm en ppb
- 0,05 : (dénominateur) prise essai de l'échantillon (ml)
- 0,1 : prise essai d'échantillon en grammes
- 130,2 : masse moléculaire du NDPA.

Les exemples suivants illustrent l'invention.

**EXEMPLE 1**

**Préparation de la nadroparine calcique purifiée avant une teneur en groupes N-NO inférieure à 100 ppb**

Stade A : Dépolymérisation et fractionnement éthanolique

Dans un réacteur on dissout 20 kg d'héparine sodique provenant de mucus intestinal de porc avec de l'eau purifiée de façon à obtenir une concentration finale voisine de 10,3 % (m/V). On ajuste le pH de la solution à 2,5 au moyen d'acide chlorhydrique concentré.
On introduit 572 g de nitrite de sodium dans le réacteur en maintenant le pH à 2,5 au moyen d'acide chlorhydrique concentré.

On suit la réaction de dépolymérisation à l'aide du papier test à l'iodure de potassium amidonné. La réaction est terminée lorsque le test est négatif.

On ajuste le pH de la solution réactionnelle à 10 avec de l'hydroxyde de sodium concentré, puis on ajoute 200 g de borohydrure de sodium. On agite pendant 15 heures puis on ajuste le pH entre 4 et 3,5 à l'aide d'acide chlorhydrique concentré, afin de détruire l'excès de borohydrure. Puis on ajuste le pH à 7 par addition d'hydroxyde de sodium concentré. On précipite ensuite en ajoutant 2 volumes d'éthanol par volume de solution aqueuse. On laisse décanter puis on élimine le surnageant hydroalcoolique. On dissout le précipité dans 400 l d'eau purifiée. On ajoute à cette solution du chlorure de sodium jusqu'à l'obtention d'une conductivité voisine de 20000 µS/cm. On ajuste ensuite le pH à une valeur voisine de 4 avec de l'acide chlorhydrique concentré et on ajoute à la solution sous agitation 1 volume d'éthanol absolu. On laisse décanter pendant 60 heures environ, puis on élimine le surnageant hydroalcoolique. On obtient ainsi la nadroparine sous forme de sel de sodium.

On dissout ensuite le précipité dans l'eau purifiée de façon à obtenir une solution à environ 18 % m/V sur la base de la quantité d'héparine sodique engagée au départ, on ajuste le pH à 7 à l'aide d'hydroxyde de sodium concentré. On filtre ensuite la solution sur un système équipé de cartouches filtrantes de porosité de 0,2 µm. On prélève de la solution filtrée contenant la nadroparine sodique un échantillon qui ne sera pas traité selon le procédé décrit au stade B (traitement par le rayonnement UV). Cet échantillon est appelé "Témoin".

Stade B : Traitement par le rayonnement UV

Pour le traitement par le rayonnement UV, on utilise un tube Katadyn de type JR1-50 de volume utile de 750 ml. La longueur d'onde utilisée est de 254 nm. On règle préalablement à l'aide d'une pompe péristaltique le débit de passage en utilisant de l'eau purifiée, de manière adéquate (4800 ml/heure) pour obtenir par un passage sans recyclage un temps d'exposition total aux UV d'environ 9 minutes. On introduit ensuite la solution de nadroparine sodique à traiter et on lave le circuit avec de l'eau purifiée jusqu'à récupération complète du produit traité dans la cuve qui est adaptée à la sortie du tube Katadyn JR1-50.

Stade C : Purification par chromatographie et transformation en sel calcique

On purifie la solution de nadroparine obtenue au stade précédent sur colonne anionique (0,5 l/kg d'héparine sodique mis en oeuvre). On ajuste la conductivité des effluents collectés à 10000-20000 µS/cm avec du chlorure de sodium et on ajoute ensuite 1,5 volume d'éthanol. On laisse décanter environ 41 heures puis on élimine le surnageant.

On dissout le précipité dans de l'eau purifiée (C = 18 % m/V sur la base de la quantité d'héparine sodique mise en oeuvre) et on ajoute du chlorure de calcium hexahydraté (9,63 g/g d'héparine sodique engagé). On procède ensuite à une précipitation éthanolique en ajoutant 1,5 volume d'éthanol. On répète l'étape de salification par le chlorure de calcium hexahydraté et l'étape de purification par précipitation éthanolique. Le précipité obtenu est séché à une température ne dépassant pas 60°C.

On obtient ainsi le lot 1.

Le lot "témoin" obtenu au Stade A est traité aussi selon le procédé décrit dans ce Stade C et on isole le lot "témoin" de nadroparine calcique.

Sur un échantillon du lot 1 et du lot "témoin"de nadroparine calcique, on effectue des analyses physicochimiques et des dosages pour déterminer leur activité biologique.

Les différents résultats sont donnés dans les tableaux I et II.

TABLEAU I

| Essais physicochimiques | | |
|---|---|---|
| **NATURE DU CONTROLE** | **LOT 1 (TRAITEMENT PAR UV)** | **TEMOIN (SANS TRAITEMENT UV)** |
| **Groupes N-NO** | 53 ppb | 3150 ppb |
| **Sulfates libres** | 0,05 % | 0,06 % |
| **pH solution à 5 %** | 6,0 | 6,0 |
| **NH$_2$ libres** | < 30 ppm | < 30 ppm |
| **HPLC-GPC (UV 205 nm) Masse moléculaire moyenne en poids (Mw)** | 5017 Da | 4933 Da |

TABLEAU I   (suite)

| Essais physicochimiques | | |
|---|---|---|
| NATURE DU CONTROLE | LOT 1 (TRAITEMENT PAR UV) | TEMOIN (SANS TRAITEMENT UV) |
| Masse moléculaire moyenne en nombre (Mn) | 4043 Da | 4052 Da |
| Poids moléculaire au sommet | 4181 Da | 4192 Da |
| Dispersion | 1,24 | 1,21 |
| % PM > 10000 Da | 2,4 | 2,1 |
| % PM > 8000 Da | 7,2 | 6,9 |
| % PM < 2000 Da | 3,0 | 2,9 |
| % PM < 1800 Da | 2,4 | 2,3 |

TABLEAU II

| Essais biologiques | | |
|---|---|---|
| NATURE DU CONTROLE | LOT 1 (TRAITEMENT PAR UV) | TEMOIN (SANS TRAITEMENT UV) |
| Activité anti-facteur Xa | 124 UI/mg | 123 UI/mg |
| Activité anti-facteur IIa | 29,3 UI/mg | 30,4 UI/mg |

Les résultats indiqués dans les tableaux I et II démontrent que le traitement par UV ne provoque aucune altération de la nadroparine. En effet les différentes caractéristiques physicochimiques et biologiques du lot 1 tels que le profil chromatographique du produit, le taux des différentes impuretés, l'activité anti-facteur Xa et l'activité anti-facteur IIa sont identiques à celles du témoin non traité par le rayonnement UV à une seule exception. Le taux des groupes N-NO du témoin non traité est environ 60 fois supérieur à celui des groupes N-NO du lot 1 qui a subit un traitement par le rayonnement UV.

Par ailleurs, les résultats d'analyses par résonance paramagnétique électronique ont confirmé que le traitement par le rayonnement UV ne provoque pas la libération de radicaux libres.

**EXEMPLE 2**

**Préparation du CY 222 sel de sodium ayant un taux de groupes N-NO inférieur à 50 ppb**

Stade A : Dépolymérisation

Dans un réacteur, on dissout 250 g d'héparine sodique provenant de mucus intestinal de porc avec de l'eau purifiée, de façon à obtenir une concentration finale voisine de 10,3 % (m/V). On ajuste le pH à 2,5 au moyen d'acide chlorhydrique concentré. On introduit 9,49 g de nitrite de sodium dans le réacteur en maintenant le pH à 2,5. On suit la réaction de dépolymérisation à l'aide du papier test à l'iodure de potassium amidonné. La réaction est terminée lorsque le test est négatif. On ajuste le pH de la solution réactionnelle entre 10 et 10,5 avec de l'hydroxyde de sodium concentré puis, on ajoute 2,5 g de borohydrure de sodium. On laisse le mélange sous agitation pendant 15 heures, puis on ajuste le pH entre 3,5 et 4 à l'aide d'acide chlorhydrique concentré afin de détruire l'excès de borohydrure. On ajuste le pH à 7 par addition d'hydroxyde de sodium concentré. On précipite ensuite en ajoutant 2 volumes d'éthanol par volume de solution. On laisse décanter le précipité et on élimine le surnageant. On obtient ainsi le CY 222 sous forme de sel de sodium. Le produit ainsi obtenu contient entre 3.000 et 10.000 ppb de groupes N-NO (Résultats de 3 préparations).

Stade B : Traitement par le rayonnement UV

On dissout le précipité de l'étape précédente dans de l'eau purifiée de façon à obtenir une solution finale à environ 10 % m/V sur la base de la quantité d'héparine sodique engagée, on ajuste le pH à 7 par de l'acide chlorhydrique ou de l'hydroxyde de sodium. Après avoir réglé le débit de la pompe, on fait passer la solution ainsi obtenue, sous un système à rayonnement ultraviolet à 254 nm (système Katadyn JR1-50 muni d'une lampe de 16 W). La durée de l'exposition au rayonnement UV est de 9 à 15 minutes.

Stade C : Purification par chromatographie et précipitations finales

On purifie la solution obtenue après traitement par rayonnement UV sur une colonne chromatographique contenant au minimum 2 litres de résine anionique par kg d'héparine sodique engagé. On ajuste la conductivité des effluents collectés à 30-35 mS/cm à l'aide de chlorure de sodium et on ajuste leur pH à 7 à l'aide d'acide chlorhydrique. Puis, on ajoute 2 volumes d'éthanol par volume de solution aqueuse. On laisse décanter le précipité et on élimine le surnageant. On dissout le précipité dans l'eau purifiée de façon à obtenir une solution finale à 20 % m/V sur la base de la quantité d'héparine sodique engagée au départ, on ajuste la conductivité de la solution à 30-35 mS/cm avec du chlorure de sodium et le pH entre 7-7,5 à l'aide de l'acide chlorhydrique concentré ou de l'hydroxyde de sodium concentré. On précipite ensuite la solution par 2 volumes d'éthanol absolu et on laisse décanter. On recueille le précipité, on le lave à l'éthanol et on le sèche à une température ne dépassant pas 60°C. On obtient ainsi le CY 222 pur, défini comme sel de sodium d'une héparine dépolymérisée obtenue par dépolymérisation à l'acide nitreux de l'héparine de mucus intestinale de porc ayant:

- une masse moléculaire majoritaire s'étageant entre 1.700 Da et 3.300 Da, 90% des constituants s'étageant entre 1.000 Da et 8.000 Da,
- une structure 2-O-sulfo-$\alpha$-L-idopyranosuronique à l'extrémité non réductrice et une structure 6-O-sulfo-2,5-anhydro-D-mannitol à l'extrémité réductrice de la majorité de ses constituants,
- un degré de sulfatation d'environ 2,1,
- une activité anti-facteur Xa de 60-80 UI/mg,
- une activité anti-facteur IIa pas supérieure à 25 UI/mg et notamment de 10-15 UI/mg,
- un contenu en groupes N-NO inférieur à 50 ppb.

**Revendications**

1. Fraction d'héparine obtenue par dépolymérisation nitreuse ayant un taux en groupes N-NO inférieur ou égal à 500 ppb.

2. Fraction d'héparine selon la revendication 1, caractérisée en ce qu'elle est une héparine de bas poids moléculaire .

3. Héparine de bas poids moléculaire selon la revendication 2, caractérisée en ce qu'elle est une héparine dépolymérisée, obtenue par dépolymérisation nitreuse d'héparine d'origine naturelle provenant de mucus intestinal de porc, du poumon de boeuf ou toute autre héparine extraite des tissus ou des organes d'animaux divers ayant les caractéristiques suivantes :

   - une masse moléculaire moyenne inférieure à 8.000 Da,
   - au moins 60 % de tous les constituants ont une masse moléculaire moyenne inférieure à 8.000 Da,
   - une activité anti-facteur Xa pas inférieure à 60 UI/mg,
   - un rapport activité anti-facteur Xa/anti-facteur 11a pas inférieur à 1,5 sous forme de sel pharmaceutiquement acceptable.

4. Héparine de bas poids moléculaire selon la revendication 3, sous forme de sel de sodium ou de sel de calcium.

5. Sel de sodium d'une héparine dépolymérisée obtenue par dépolymérisation à l'acide nitreux de l'héparine d'origine naturelle de mucus intestinal de porc ayant :

   - une masse moléculaire majoritaire s'étalant entre 1.700 Da et 3.300 Da,
   - 90 % des constituants ayant une masse moléculaire s'étalant entre 1.000 Da et 8.000 Da,
   - une structure 2-O-sulfo-$\alpha$-L-idopyranosuronique à l'extrémité non réductrice et une structure 6-O-sulfo-2,5-anhydro-D-mannitol à l'extrémité réductrice de la majorité de ses constituants,
   - un degré de sulfatation d'environ 2,1,
   - une activité anti-facteur Xa de 60-80 UI/mg,
   - une activité anti-facteur 11a pas supérieure à 25 UI/mg,
   - un taux en groupes N-NO inférieur ou égal à 100 ppb.

6. Sel de sodium d'une héparine dépolymérisée selon la revendication 5, caractérisé en ce que le taux en groupes N-NO est inférieur ou égal à 50 ppb.

**7.** Procédé pour la préparation d'une fraction d'héparine selon l'une quelconque des revendications 1 à 6, caractérisé en ce qu'on soumet une solution aqueuse d'une fraction d'héparine obtenue par dépolymérisation nitreuse à un rayonnement ultraviolet.

**8.** Procédé selon la revendication 7, caractérisé en ce qu'on expose une solution aqueuse d'une fraction d'héparine obtenue par dépolymérisation nitreuse de concentration de 5-15 % m/V, sous un système à rayonnement ultraviolet à une longueur d'onde de 180 à 350 nm, à une température comprise entre 5°C et 50°C, le pH de la solution étant entre 3 et 8.

**9.** Procédé selon l'une des revendications 7 ou 8, caractérisé en ce qu'on opère à 254 nm, à une température comprise entre 15°C et 35°C et à un pH entre 5 et 8.

**10.** Procédé pour la préparation d'une héparine de bas poids moléculaire selon l'une quelconque des revendications 1 à 5, caractérisé en ce que:

(a) on traite une solution d'héparine sodique non fractionnée dans l'eau à 5-15 % m/V avec une solution de nitrite de sodium, à raison de 15 à 69 g de nitrite par kg d'héparine mise en oeuvre, en présence d'acide chlorhydrique à un pH de 1 à 5, de préférence 1,5 à 4, pour une durée de 20 à 50 minutes et on soumet le produit ainsi obtenu à une réduction, de préférence en milieu alcalin, avec 5-20 g de borohydrure de sodium par kg d'héparine mise en oeuvre au départ, on détruit l'excès de borohydrure de sodium par l'acide chlorhydrique, on neutralise éventuellement le milieu réactionnel, et on précipite à l'éthanol, puis après avoir éventuellement soumis l'héparine de bas poids moléculaire ainsi obtenue à un ou plusieurs fractionnements alcooliques,
(b) on soumet la solution ainsi obtenue à un rayonnement ultraviolet,
(c) on procède éventuellement à une purification chromatographique et on isole l'héparine sodique dépolymérisée, purifiée et exempte de groupes N-NO par précipitation avec du chlorure de sodium et de l'éthanol, et, éventuellement,
(d) on transforme le sel de sodium en un autre sel pharmaceutiquement acceptable.

**11.** Procédé pour la préparation du produit selon la revendication 6, caractérisé en ce que :

(a) on traite une solution aqueuse d'héparine sodique non fractionnée avec de l'acide chlorhydrique et une quantité d'un nitrite alcalin de 3,5 à 4% en poids par rapport à l'héparine mise en oeuvre, tout en maintenant le pH acide et en surveillant la présence d'ions nitreux jusqu'à réaction négative, puis on alcalinise, on réduit avec du borohydrure de sodium et on isole en milieu neutre le produit dépolymérisé par précipitation par l'éthanol, puis,
(b) on fait passer une solution aqueuse du produit ainsi obtenu, à pH d'environ 7, sous un système à rayonnement ultraviolet à 254 nm, on introduit ensuite la solution ainsi obtenue au sommet d'une colonne échangeuse d'anions et, après rinçage de la colonne à l'eau et à pH d'environ 7, on récupère le produit final par précipitation avec du chlorure de sodium et d'éthanol.

**12.** Procédé selon la revendication 11, caractérisé en ce que dans l'étape (b), on utilise un système à rayonnement ultraviolet à 254 nm pendant 9 à 15 minutes avec une lampe UV de 16 W, la concentration de la solution du produit soumis au rayonnement UV étant de 8-12 % m/V.

**13.** Composition pharmaceutique contenant, en tant que principe actif, une fraction d'héparine selon l'une quelconque des revendications 1 à 6.

**14.** Composition pharmaceutique contenant comme principe actif, 10 mg à 5.000 mg du produit selon la revendication 6, par unité de dosage.

**Patentansprüche**

**1.** Durch Nitrit-Depolymerisation erhaltene Heparin-Fraktion mit einem Gehalt an Gruppen N-NO von höchstens 500 ppb.

**2.** Heparin-Fraktion nach Anspruch 1, dadurch gekennzeichnet, daß sie ein Heparin mit niedriger Molekülmasse darstellt.

3. Heparin mit niedrigem Molekulargewicht nach Anspruch 2, dadurch gekennzeichnet, daß es ein depolymerisiertes Heparin ist, das durch Nitrit-Depolymerisation eines natürlichen Heparins aus der Darmschleimhaut von Schweinen oder Rinderlunge oder eines beliebigen anderen Heparins, das aus Gewebe oder Organen von davon verschiedenen Tieren gewonnen wurde, erhalten ist und folgende Eigenschaften aufweist:

- die mittlere Molekülmasse ist kleiner als 8000 Da,
- mindestens 60 % aller Bestandteile weisen eine mittlere Molekülmasse von weniger als 8000 Da auf,
- die Anti-Faktor-Xa-Aktivität ist nicht geringer als 60IU/mg,
- das Verhältnis von Anti-Faktor-Xa-Aktivität zu Anti-Faktor-IIa-Aktivität ist nicht kleiner als 1,5, in Form eines pharmazeutisch akzeptablen Salzes.

4. Heparin mit niedriger Molekülmasse nach Anspruch 3 in Form des Natrium- oder Calciumsalzes.

5. Natriumsalz eines depolymerisierten Heparins, das durch Depolymerisation eines natürlichen Heparins aus der Darmschleimhaut von Schweinen mit salpetriger Säure erhalten wurde, mit folgenden Eigenschaften:

- die überwiegende Molekülmasse liegt im Bereich von 1700 bis 3300 Da,
- 90 % der Bestandteile besitzen eine Molekülmasse im Bereich von 1000 bis 8000 Da,
- der überwiegende Teil der Bestandteile weist am nicht-reduzierenden Ende eine 2-0-Sulfo-$\alpha$-L-idopyranosuronsäure-Struktur und am reduzierenden Ende eine 6-0-Sulfo-2,5-anhydro-D-mannit-Struktur auf,
- der Sulfatierungsgrad beträgt etwa 2,1,
- die Anti-Faktor-Xa-Aktivität beträgt 60 bis 80IU/mg,
- die Anti-Faktor-IIa-Aktivität ist nicht größer als 25 IU/mg,
- der Gehalt an Gruppen N-NO ist höchstens gleich 100 ppb.

6. Natriumsalz eines depolymerisierten Heparins nach Anspruch 5, dadurch gekennzeichnet, daß der Gehalt an Gruppen N-NO höchstens gleich 50 ppb ist.

7. Verfahren zur Herstellung einer Heparin-Fraktion nach einem der Ansprüche 1 bis 6, dadurch gekennzeichnet, daß eine wäßrige Lösung einer durch Nitrit-Depolymerisation erhaltenen Heparin-Fraktion mit UV-Strahlung bestrahlt wird.

8. Verfahren nach Anspruch 7, dadurch gekennzeichnet, daß eine wäßrige Lösung einer durch Nitrit-Depolymerisation erhaltenen Heparin-Fraktion einer Konzentration von 5 bis 15 % (M/V) mit einem UV-Bestrahlungssystem bei einer Wellenlänge von 180 bis 350 nm bei einer Temperatur im Bereich von 3 bis 8 bestrahlt wird.

9. Verfahren nach Anspruch 7 oder 8, dadurch gekennzeichnet, daß bei einer Wellenlänge von 254 nm einer Temperatur im Bereich von 15 bis 35 °C und einem pH-Wert im Bereich von 5 bis 8 gearbeitet wird.

10. Verfahren zur Herstellung eines Heparins mit niedriger Molekülmasse nach einem der Ansprüche 1 bis 5, gekennzeichnet durch folgende Schritte:

(a) Behandlung einer Lösung von nichtfraktioniertem Heparin-Natrium in Wasser einer Konzentration von 5 bis 15 % (M/V) mit einer Natriumnitrit-Lösung in einer Menge von 15 bis 69 g Nitrit pro Kilogramm eingesetzten Heparins in Gegenwart von Salzsäure bei einem pH-Wert von 1 bis 5 und, vorzugsweise 1,5 bis 4 während einer Dauer von 20 bis 50 min, Reduktion des so erhaltenen Produkts, vorzugsweise in alkalischem Medium, mit 5 bis 20 g Natriumborhydrid pro Kilogramm anfänglich eingesetzten Heparins, Vernichten von überschüssigem Natriumborhydrid mit Salzsäure, ggf. Neutralisation des Reaktionsmediums, sowie Fällung mit Ethanol und ggfs. einmalige oder mehrmalige Fraktionierung des so erhaltenen Heparins mit niedrigem Molekülgewicht mit Alkohol,
(b) Bestrahlung der so erhaltenen Lösung mit UV-Strahlung,
(c) ggfs. chromatographische Reinigung und Isolierung des depolymerisierten greinigten Heparin-Natriums ohne Gruppen N-NO durch Fällung mit Natriumchlorid, Ethanol und ggfs.
(d) Überführung des Natriumsalzes in ein anderes pharmazeutisch akzeptables Salz.

11. Verfahren zur Herstellung des Produkts nach Anspruch 6, gekennzeichnet durch folgende Schritte:

(a) Behandlung einer wäßrigen Lösung von nichtfraktioniertem Heparin-Natrium mit Salzsäure und einem Alkalinitrit in einer Menge von 3,5 bis 4 Gew.-%, bezogen auf das eingesetzte Heparin, wobei der pH-Wert sauer gehalten wird und das Vorliegen von Nitritionen bis zur negativen Reaktion überwacht wird, anschließendes Alkalischmachen/-Reduktion mit Natriumborhydrid und Isolierung des depolymerisierten Produkts durch Ausfällung mit Ethanol in neutralem Medium und anschließendes
(b) Vorbeileiten einer wäßrigen Lösung des so erhaltenen Produkts bei einem pH-Wert von etwa 7 an einem UV-Bestrahlungssystem von 254 nm, anschließende Einführung der so erhaltenen Lösung oben in eine Ionenaustauschersäule und Gewinnung des Endprodukts nach Spülen der Säule mit Wasser bei einem pH-Wert von etwa 7 durch Ausfällung mit Natriumchlorid und Ethanol.

12. Verfahren nach Anspruch 11, dadurch gekennzeichnet, daß in Schritt (b) ein UV-System mit einer Wellenlänge von 254 nm und einer UV-Lampe von 16 W verwendet und eine Bestrahlungsdauer von 9 bis 15 min angewandt wird, wobei die Konzentration der mit UV bestrahlten Produktlösung im Bereich von 8 bis 12 % (M/V) liegt.

13. Pharmazeutische Zusammensetzung, die eine Heparin-Fraktion nach einem der Ansprüche 1 bis 6 als Wirkstoff enthält.

14. Pharmazeutische Zusammensetzung, die als Wirkstoff 10 bis 5000 mg des Produkts nach Anspruch 6 pro Dosiereinheit enthält.


## Claims

1. Heparin fraction obtained by nitrous depolymerization having a content of N-NO compounds not exceeding 500 ppb.

2. Heparin fraction according to Claim 1, characterized in that it is a low-molecular-weight heparin.

3. Low-molecular-weight heparin according to Claim 2, characterized in that it is a depolymerized heparin obtained by nitrous depolymerization of heparin of natural origin originating from porcine intestinal mucosa or from bovine lung or any other heparin extracted from tissues or organs of various animals, having the following characteristics:

   - an average molecular mass of less than 8,000 Da,
   - at least 60 % of all the constituents have an average molecular mass of less than 8,000 Da,
   - an anti-factor Xa activity not less than 60 IU/mg,
   - an anti-factor Xa/anti-factor IIa activity ratio not less than 1.5, in the form of a pharmaceutically acceptable salt.

4. Low-molecular-weight heparin according to Claim 3, in the form of a sodium salt or calcium salt.

5. Sodium salt of a depolymerized heparin obtained by nitrous acid depolymerization of heparin of natural origin from porcine intestinal mucosa, having:

   - a preponderant molecular mass ranging between 1,700 Da and 3,300 Da,
   - 90 % of the constituents having a molecular mass ranging between 1,000 Da and 8,000 Da,
   - a 2-O-sulpho-$\alpha$-L-idopyranosuronic structure at the non-reducing end and a 6-O-sulpho-2,5-anhydro-D-mannitol structure at the reducing end of the majority of its constituents,
   - a degree of sulphation of approximately 2.1,
   - an anti-factor Xa activity of 60-80 IU/mg,
   - an anti-factor IIa activity not greater than 25 IU/mg,
   - a content of N-NO compounds not exceeding 100 ppb.

6. Sodium salt of a depolymerized heparin according to Claim 5, characterized in that the content of N-NO compounds does not exceed 50 ppb.

7. Method for the preparation of a heparin fraction according to any one of Claims 1 to 6, characterized in that an aqueous solution of a heparin fraction obtained by nitrous depolymerization is subjected to ultraviolet radiation.

8. Method according to Claim 7, characterized in that an aqueous solution of a heparin fraction obtained by nitrous depolymerization, of concentration 5-15 % m/V, is exposed under an ultraviolet radiation system at a wavelength of 180 to 350 nm, at a temperature of between 5°C and 50°C, the pH of the solution being between 3 and 8.

9. Method according to one of Claims 7 and 8, characterized in that exposure takes place at 254 nm, at a temperature of between 15°C and 35°C and at a pH of between 5 and 8.

10. Method for the preparation of a low-molecular-weight heparin according to any one of Claims 1 to 5, characterized in that:

(a) a 5-15 % m/V solution of unfractionated heparin sodium in water is treated with a solution of sodium nitrite, in the proportion of 15 to 69 g of nitrite per kg of heparin introduced, in the presence of hydrochloric acid at a pH of 1 to 5, and preferably 1.5 to 4, for a period of 20 to 50 minutes, and the product thereby obtained is subjected to a reduction, preferably in an alkaline medium, with 5-20 g of sodium borohydride per kg of heparin introduced at the start, the excess sodium borohydride is destroyed with hydrochloric acid, the reaction medium is neutralized where appropriate and the product is precipitated with ethanol, then, after the low-molecular-weight heparin thereby obtained has been subjected, where appropriate, to one or more alcohol fractionations,
(b) the solution thereby obtained is subjected to ultraviolet radiation,
(c) a chromatographic purification is performed where appropriate and the depolymerized heparin sodium, purified and free from N-NO compounds, is isolated by precipitation with sodium chloride and ethanol and, where appropriate,
(d) the sodium salt is converted to another pharmaceutically acceptable salt.

11. Method for the preparation of the product according to Claim 6, characterized in that:

(a) an aqueous solution of unfractionated heparin sodium is treated with hydrochloric acid and an amount of an alkali metal nitrite of 3.5 to 4 % by weight relative to the heparin introduced, while the pH is kept acidic and the presence of nitrous ions is monitored until there is a negative reaction, the mixture is then alcalinized, the product is reduced with sodium borohydride and the depolymerized product is isolated in a neutral medium by precipitation with ethanol, then
(b) an aqueous solution of the product thereby obtained is passed at a pH of approximately 7 under an ultraviolet radiation system at 254 nm, the solution thereby obtained is thereafter introduced at the top of an anion exchange column and, after the column is rinsed with water and at a pH of approximately 7, the final product is recovered by precipitation with sodium chloride and ethanol.

12. Method according to Claim 11, characterized in that, in step (b), an ultraviolet radiation system at 254 nm is used for 9 to 15 minutes with a 16 W UV lamp, the concentration of the solution of the product subjected to UV radiation being 8-12 % m/V.

13. Pharmaceutical composition containing as active principle a heparin fraction according to any one of Claims 1 to 6.

14. Pharmaceutical composition containing as active principle 10 mg to 5,000 mg of the product according to Claim 6 per dosage unit.

# FIG.1

FIG.2